(19) **Europäisches Patentamt / European Patent Office / Office européen des brevets**

(11) **EP 4 230 141 A1**

(12) **DEMANDE DE BREVET EUROPEEN**

(43) Date de publication:
23.08.2023  Bulletin 2023/34

(21) Numéro de dépôt: 22306332.2

(22) Date de dépôt: 08.09.2022

(51) Classification Internationale des Brevets (IPC):
*A61B 5/397* (2021.01)    *A61B 5/00* (2006.01)

(52) Classification Coopérative des Brevets (CPC):
**A61B 5/397; A61B 5/725; A61B 5/7253; A61B 5/7267**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**
Etats de validation désignés:
**KH MA MD TN**

(30) Priorité:  21.02.2022  EP 22305187

(71) Demandeur: **Blueback**
**35510 Cession-Sévigné (FR)**

(72) Inventeurs:
• **MOUDJARI, Inès**
  **06610 LA GAUDE (FR)**
• **PAUTARD, Caroline**
  **35850 PARTHENAY DE BRETAGNE (FR)**
• **JOUANNEAU, Clément**
  **35850 PARTHENAY DE BRETAGNE (FR)**

(74) Mandataire: **Novagraaf Technologies**
**Bâtiment O2**
**2, rue Sarah Bernhardt**
**CS90017**
**92665 Asnières-sur-Seine Cedex (FR)**

(54) **MÉTHODE IMPLÉMENTÉE PAR ORDINATEUR DE CLASSIFICATION DE SIGNAUX PHYSIOLOGIQUES, ET UTILISATION POUR LA MESURE DE L'ACTIVITÉ MUSCULAIRE SPÉCIFIQUE D'UN GROUPEMENT DE MUSCLES D'UN SUJET**

(57)    La présente invention se rapporte à une méthode implémentée par ordinateur de classification de signaux physiologiques issus de l'activité musculaire spécifique d'un groupement de muscles comprenant au moins un muscle profond et au moins un muscle de surface d'un sujet.

La présente invention se rapporte également à une méthode de mesure de l'activité musculaire spécifique d'un groupement de muscles comprenant au moins un muscle profond et au moins un muscle de surface d'un sujet

La présente invention se rapporte en outre à une utilisation de la méthode de mesure pour une application choisie parmi la rééducation fonctionnelle, le renforcement musculaire à des fins de bien-être et/ou esthétiques, la prévention et le diagnostic fonctionnel, ainsi qu'à un produit programme d'ordinateur.

## Description

### Domaine technique

La présente invention se rapporte à une méthode implémentée par ordinateur de classification de signaux physiologiques issus de l'activité musculaire spécifique d'un groupement de muscles comprenant au moins un muscle profond et au moins un muscle de surface d'un sujet, ainsi qu'à une méthode de mesure de l'activité musculaire spécifique d'un groupement de muscles comprenant au moins un muscle profond et au moins un muscle de surface d'un sujet , à son utilisation et à un produit programme d'ordinateur.

La présente invention trouve des applications notamment dans les domaines de la rééducation fonctionnelle, le sport, le bien-être, l'esthétique ou encore le diagnostic fonctionnel.

Dans la description ci-dessous, les références entre crochets ([]) renvoient à la liste des références présentée à la fin du texte.

### Etat de la technique

Dans le domaine de la mesure non invasive de l'activité musculaire, on connaît la mesure par électromyographie (EMG), consistant à positionner des électrodes adhésives sur la peau du patient, stratégiquement par rapport à l'anatomie du muscle pour lequel on cherche à enregistrer l'activité musculaire.

Cette technique est limitée car le signal recueilli ne peut être spécifique du muscle ciblé que si ledit muscle est positionné juste en dessous de l'électrode de mesure. Elle s'applique donc principalement pour les muscles dits superficiels, situés juste en dessous de la peau. Le projet SENIAM (http://www.seniam.org/) a d'ailleurs été conduit dans le but de consolider la recherche appliquée dans le domaine de l'électromyographie de surface, ce qui a résulté en la publication de recommandations européennes concernant les capteurs, leur positionnement optimal par muscle ciblé et les méthodes de traitement de signal adaptées. Ces recommandations concernent donc 30 muscles individuels, mais ne comprennent pas les muscles profonds. De même, il y a une dichotomie entre le nombre de capteur et le nombre de muscles que l'on souhaite caractériser: si on souhaite connaître l'activité musculaire spécifique d'un muscle, il faut au moins un capteur. Un même capteur ne pourra servir à caractériser plus d'un muscle de façon spécifique. Si un capteur est utilisé pour caractériser plus d'un muscle de surface, l'information recueillie est donc combinée, c'est à dire que l'on sait que l'un des muscles est contracté, ou que tous les muscles sont relâchés, mais aucune information supplémentaire ne peut en être déduite.

Parallèlement, il y a eu plusieurs tentatives pour mesurer l'activité musculaire de muscles profonds à partir d'électrodes EMG de surface.

McGill et al (1996, J. Biomechanics, Vol 29, No 11, pp 1503-1507 ([1])) ont étudié la possibilité de mesurer l'activité musculaire de 5 muscles spécifiques (le psoas, l'oblique externe, l'oblique interne, le transverse abdominal et le carré de lombes) à partir d'une unique électrode EMG de surface positionnée de façon stratégique pour chaque muscle. Cette étude montre que certains sites anatomiques de surface pourraient être utilisés pour refléter l'activité musculaire de muscles profonds comme le carré des lombes et le psoas. Cependant, l'erreur sur la mesure est de 6% à 12%. De plus, les conclusions montrent que la localisation qui permettrait de mesurer (avec une erreur de 15%) le transverse abdominal est la même que celle qui représente au mieux l'activité musculaire de l'oblique interne (15cm en latéral du nombril, juste au-dessus du ligament inguinal). Ainsi, cette localisation n'est pas spécifique d'un de ces deux muscles et la méthode ainsi décrite ne permet pas de mesurer l'activité musculaire spécifique d'un groupement de muscles comprenant au moins un muscle profond et au moins un muscle de surface.

Jesinger et Stonick (1994, 6th IEEE DSP Workshop Proc., p57-60 ([2])) proposent de résoudre un problème inverse basé sur une modélisation en éléments finis de l'activité électrique des muscles profonds et de sa propagation jusqu'à la surface de la peau. Cette méthode, comme d'autres méthodes basées sur des principes similaires de résolution inverse issues d'un modèle, nécessite un nombre élevé d'électrodes (plus de 100) pour obtenir la quantité d'information suffisante permettant de résoudre le problème mathématique. Ces méthodes sont d'ailleurs principalement utilisées à des fins de recherche et ne sont pas nécessairement adaptées à des applications industrielles pour lesquelles les calculs doivent être faits parfois en temps réel, et avec un dispositif simple d'utilisation.

L'invention décrite dans le document US 9,687,168 ([3]) consiste en une méthode d'électromyographie de surface des muscles profonds. L'invention consiste à disposer une matrice d'électrodes mono-polaires encerclant la circonférence d'une partie du corps dans laquelle est incluse le muscle profond que l'on cherche à investiguer. L'exemple d'application retenu concerne la mesure du muscle brachial du bras, en disposant de deux matrices de 6 électrodes chacune, configurées en deux cercles concentriques autour du bras. Cette méthode doit donc utiliser 12 électrodes pour caractériser un muscle. De plus, ce brevet n'enseigne pas le mode de réalisation permettant d'appliquer la méthode pour les muscles profonds sur d'autres zones anatomiques, étant donné qu'une phase de calibration spécifique de la distribution des muscles de la zone investiguée est nécessaire à l'application de l'analyse en composants indépendants réalisée par la suite.

Enfin, le document WO2019097166 ([4]) concerne un dispositif de mesure de l'activité musculaire spécifique d'un ou plusieurs muscles profonds de la sangle abdominale d'un sujet à partir de capteurs EMG de surface, permettant de mesurer l'activité musculaire de plusieurs muscles profonds (notés N) simultanément, tout en obtenant un résultat spécifique, c'est à dire individualisée, l'activité soit d'un muscle profond, ou bien de plusieurs muscles profonds en, même temps, tout en pouvant attribuer spécifiquement à chacun des muscles la valeur de l'activité musculaire mesurée pour ce muscle. Dans cette invention, le dispositif ne permet pas de mesurer de façon spécifique et individualisée, l'activité musculaire d'un ensemble de muscles comprenant également des muscles de surface. De plus, l'invention concerne les muscles profonds de la sangle abdominale uniquement, sans pouvoir être transposable à un autre site anatomique.

Il existe par ailleurs des méthodes basées sur la mise au point et le paramétrage des classifieurs de signaux.

Le document Fajardo et al. (« EMG hand gesture classification using handcrafted and deep features », Biomedical Signal Processing and Control, Volume 63, January 2021, 102210 ([5])) décrit un procédé mettant en oeuvre un classifieur de type MLP (multi-layer perceptron) permettant de classer des signaux EMG en fonction du geste réalisé par une main. Cette méthode ne permet pas de différencier l'activité musculaire de chaque muscle de la zone, de façon spécifique et indépendante.

Le document Arteaga et al. (« EMG-driven hand model based on the classification of individual finger movements », Biomedical Signal Processing and Control, Volume 58, April 2020, 101834 ([6])) présente un procédé mettant en œuvre un classifieur de type SVM (Support Vector Machines) permettant de classer des signaux EMG en fonction du geste réalisé par la main et des mouvements des doigts. Cette méthode ne permet pas de différencier l'activité musculaire de chaque muscle de la zone, de façon spécifique et indépendante.

Il existe donc un réel besoin d'une méthode permettant de mesurer l'activité musculaire spécifique d'un groupement de muscles comprenant au moins un muscle profond et au moins un muscle de surface.

**Description de l'invention**

La présente invention a précisément pour but de répondre à ces besoins et inconvénients en fournissant une méthode permettant de mesurer l'activité musculaire spécifique d'un groupement de muscles comprenant au moins un muscle profond et au moins un muscle de surface.

En effet, au terme d'importantes recherches, la Demanderesse a réussi à mettre au point une méthode permettant de caractériser les schémas de co-contraction musculaires dans une zone anatomique donnée, à partir uniquement de capteurs de surface, pouvant être non invasifs. En outre, ce groupement de muscles contient au moins un muscle profond et au moins un muscle de surface.

Avantageusement, la méthode de l'invention permet de prendre des mesures sur un site anatomique, puis d'optimiser et de paramétrer une méthode de classification des signaux recueillis, ceci permettant d'obtenir un classifieur. Le classifieur pourra par la suite ranger tout autre signal, par exemple recueilli par des capteurs non invasifs comme des EMG de surface, avec un nombre restreint de capteurs, dans des classes différenciées par le nombre et la nature des muscles simultanément contractés pendant les mesures, la zone anatomique couverte par la mesure.

De plus, contrairement aux méthodes de l'art antérieur, la méthode de la présente invention permet, une fois le classifieur réalisé, de n'utiliser qu'un nombre restreint de capteurs, inférieur au nombre de muscles à caractériser.

Le domaine de l'invention concerne une variété d'applications comme la rééducation fonctionnelle, les performances sportives, le bien-être et l'esthétique, ou encore la prévention et le diagnostic fonctionnel.

Ainsi, un premier objet de l'invention se rapporte à une méthode implémentée par ordinateur de classification de signaux physiologiques issus de l'activité musculaire spécifique d'un groupement de muscles comprenant au moins un muscle profond et au moins un muscle de surface d'un sujet, comprenant les étapes suivantes :

a) acquisition des signaux électriques issus d'au moins un capteur placé sur la peau d'un sujet,
b) pré-traitement des signaux électriques acquis pendant l'étape a), pour éliminer les bruits parasites et les artéfacts de mouvement du sujet,
c) extraction d'au moins une variable temporelle, au moins une variable fréquentielle, au moins une variable temps-fréquence, au moins une variable fractale, au moins une variable cepstrale et au moins une variable statistique, sur les signaux pré-traités de l'étape issus de l'étape b),
d) sélection de variables par analyse de la variance pour classifier des données ultérieures en fonction de classes choisies, les variables sélectionnées formant un classifieur,
e) implémentation du classifieur à partir des variables sélectionnées à l'étape d),
f) évaluation de la performance du classifieur par validation croisée et indicateurs de performance,
g) utilisation du classifieur pour caractériser l'activité musculaire des groupements musculaires.

Avantageusement, la méthode de l'invention peut être appliquée sur n'importe quel muscle ou groupe de muscle du corps humain ou animal. Par exemple, il peut s'agir des muscles de la sangle abdominale, du dos, des fessiers, du bras, de l'avant-bras, de la jambe, de la cuisse, de la face, du cou, du thorax, de l'épaule, du pied.

On entend par « muscle profond » au sens de la présente invention, tout muscle qui se trouve sous les couches de muscles externes et visibles. Il peut s'agir par exemple d'au moins un muscle choisi parmi le transverse abdominal, le psoas, le carré de lombes, l'oblique interne, l'ilio dorsal, le long dorsal, les intervertébraux, le sus épineux, les muscles du périnée, les multifides, les muscles de la colonne vertébrale, notamment qui relient l'apophyse épineuse et les apophyses transverses de chaque vertèbre, et la musculature autochtone du dos.

On entend par « muscle de surface », ou « muscle superficiel », au sens de la présente invention, tout muscle qui est visible sous la peau. Il peut s'agir d'au moins un muscle choisi parmi l'oblique externe, le grand droit, les muscles scalènes, le muscle sterno-cléido-mastoïdien, les trapèzes, le grand pectoral, les deltoïdes, le grand dorsal, les muscles intercostaux, les biceps, les triceps, les fléchisseurs de l'avant-bras, ou les extenseurs de l'avant-bras, les fessiers, les abducteurs, les adducteurs, les ischio-jambiers, les quadriceps et les jumeaux.

Les mesures peuvent être recueillies avant l'étape a) sur une zone anatomique à partir d'au moins un capteur permettant, à chaque instant, d'avoir une information sur la contraction ou le relâchement de chaque muscle, qu'il soit profond ou de surface. Les capteurs peuvent être positionnés directement au-dessus d'un ou plusieurs muscles de surface ou de muscles profonds à analyser, ou de part et d'autre d'un muscle de surface ou d'un muscle profond à analyser. Avantageusement, pour que la méthode de l'invention soit applicable, il suffit que la contraction de chaque muscle à analyser soit visible sur le signal recueilli par le ou les capteurs de la zone, sans qu'il soit nécessaire que cette contribution permette à elle seule de déterminer l'état de contraction dudit muscle.

Les capteurs susceptibles d'être utilisés pour la mise en œuvre de l'étape a) peuvent être tout capteur de surface, c'est-à-dire tout dispositif permettant de capter un phénomène physique et de le restituer sous forme de signal, en l'occurrence électrique (étape d'acquisition des signaux électriques). Il peut s'agir de capteurs non invasifs, par exemple à placer et/ou à coller sur la peau d'un sujet, ou de capteurs invasifs, par exemple à introduire au moins en partie dans la peau. Les capteurs non invasifs peuvent être par exemple des électrodes électromyographiques (EMG), des patchs, des tatouages temporaires électroniques ou d'imagerie médicale. Les capteurs invasifs peuvent être par exemple des patchs munis d'une aiguille touchant directement la fibre musculaire. Les capteurs sont des capteurs usuellement utilisés pour ce type de mesure, et peuvent à ce titre être munis de conducteurs flexibles les reliant individuellement à des moyens d'enregistrement et des moyens de calcul adaptés pour effectuer une analyse en composantes indépendantes. Il peut s'agir également de moyens d'imagerie usuellement utilisés pour ce type de mesure. Dans le cadre de l'invention, il est possible d'utiliser des capteurs invasifs et des capteurs non invasifs simultanément, ou bien l'une des deux sortes simultanément. Le nombre de capteurs susceptibles d'être utilisés lors de la mise en œuvre de l'étape a) est illimité.

Avantageusement, l'étape d'acquisition a) de la méthode peut permettre d'obtenir une base de données de signaux assez fournie pour servir ensuite de base d'apprentissage, et pour permettre de paramétrer de façon optimale le classifieur.

L'étape b) de pré-traitement des signaux électriques peut être toute méthode connue de l'homme du métier pour tout ou partie éliminer tout ou partie des bruits parasites et des artéfacts de mouvement du sujet. Il peut s'agir par exemple d'un filtrage de signaux, par exemple au moyen d'un filtre passe haut de fréquence de coupure entre 15 Hz et 50 Hz, afin d'éliminer la valeur moyenne des signaux et les variations de la ligne de base. Les signaux peuvent être par la suite redressés à l'aide de la valeur absolue du signal.

Lors de l'étape c) d'extraction, les variables peuvent être choisies parmi celles communément utilisées pour analyser des signaux relatifs à l'activité musculaire. Il peut s'agir par exemple de signaux couramment utilisés pour l'étude des signaux EMG, tels que :

- Des indicateurs temporels, comme :

  ○ Longueur de la forme d'onde (ou Waveform Length, ou WL) (Cengiz Tepe, Mehmet Can Demir ([9]))
  ○ La moyenne des amplitudes du signal (MAV)
  ○ La somme des amplitudes du signal temporel divisée par la valeur maximale du signal (somme(amp)/max)
  ○ La corrélation temporelle entre deux voies de mesures (corr)Paramètres de Hjorth (Hj) tels que l'activité (A), la mobilité (mobility) ou la complexité (complexity) (Jose Manuel Fajardo et al. ([10]) ; Noemi Gozzia et al. ([11]))
  ○ l'asymétrie (ou Skewness, ou Sk) (Jose Manuel Fajardo et al. ([10]) ; Noemi Gozzia et al. ([11]) ; Firas Sabar Miften et al. ([12]))

- Des indicateurs fréquentiels, comme :

  ○ Fréquence moyenne (fm) (Cengiz Tepe, Mehmet Can Demir ([9]) ; Maria V.Arteaga et al. ([13]))
  ○ Fréquence médiane (fmd) (Cengiz Tepe, Mehmet Can Demir ([9]) ; Maria V.Arteaga et al. ([13]))
  ○ La fréquence de coupure (fp). Il s'agit de la valeur de fréquence pour laquelle plus de 60% de la puissance du spectre se trouve antérieure à cette dernière, le taux de 60% pouvant être choisi entre 50% et 95%.

- Des indicateurs temps - fréquence, comme:

  ○ Décomposition en mode empirique (EMD) (Lingmei Ai et al. ([8])
  ○ fréquence moyenne immédiate DWT (FMI_dwt) (Abdoulaye Thioune ([14]))

La base de données des indicateurs formée lors de l'étape c) est également composée d'indicateurs qui sont utilisés dans d'autres domaines techniques, comme l'analyse fractale qui est depuis quelques années utilisée pour analyser des signaux de marché financier ou pour modéliser le trafic sur des réseaux informatiques. Parmi les variables fractales susceptibles d'être utilisées, on peut citer l'indicateur suivant :

- Exposant de Hurst : H (Mario Cifrek et al. ([15]))

Une autre famille d'indicateurs qui ne sont pas utilisés dans l'art antérieur pour analyser des signaux EMG est l'analyse cepstral. En effet, cette méthode est généralement utilisée pour traiter des signaux sonores. Elle est notamment utilisée pour déterminer des empreintes vocales. De ce type d'analyse, on peut extraire au moins un indicateur choisi parmi les suivants :

- Coefficient cepstraux (Cc) (Noemi Gozzia et al. ([11]))
- les calculs d'indicateurs temporels tels que la moyenne (mean), la médiane (médian), l'écart quadratique (std) ou la moyenne quadratique (rms) sur les coefficients cepstraux (Cci),
- les calculs d'indicateurs mathématiques tels que le minimum (min) et le maximum (max) sur les coefficients cepstraux (Cci)

La base de données formée lors de l'étape c) contient également des indicateurs statistiques représentant l'entropie du signal - certains sont couramment utilisé en EMG, d'autres pas. Ces indicateurs peuvent être choisis parmi :

- l'entropie de l'échantillon (ou sample entropy, ou SampEn) (Noemi Gozzia et al. ([11])),
- l'entropie floue (ou Fuzzy entropy, ou FuzzyEn) (16. Lorenz Kahl, Ulrich G. Hofmann ([16]))
- l'entropie de Shannon (Shannon entropy, ou ShanEn) (Arlene John et al. ([17]))

Avantageusement, les valeurs des variables peuvent être par la suite mises sous forme d'une matrice, avec chaque ligne représentant un signal et chaque colonne correspondant à la variable calculée sur ce signal (indicateur).

Avantageusement, une fois que les variables sont calculées sur tous les signaux, l'étape suivante, à savoir l'étape d), peut comprendre la sélection du jeu d'indicateurs qui permettra au mieux de classifier les données futures selon les classes choisies, c'est-à-dire selon des critères liés au nombre et au type de muscles contractés au même moment dans la zone anatomique couverte par la mesure. Cette sélection peut se faire selon toute méthode connue de l'homme du métier, par exemple par l'étude individuelle des variables calculées selon les indicateurs, afin de connaître leurs pouvoirs discriminants, seul et/ou en combinaison, pour l'ensemble des muscles sélectionnés et des classes souhaitées dans le futur classifieur. L'homme du métier sait choisir au moyen de ses connaissances générales une combinaison d'indicateurs en fonction des classes souhaitées. Par exemple, il est possible de choisir un indicateur ayant un bon pouvoir discriminant pour séparer deux classes, en combinaison avec un indicateur ayant un bon pouvoir discriminant pour deux autres classes par exemple ; en combinaison, ils auront alors un très bon pouvoir discriminant pour séparer les 3 classes, unes à unes. Différentes combinaisons d'indicateurs peuvent ensuite être choisies et testées au sein du classifieur pour évaluer la performance combinée du jeu choisi.

Cette étape comprend une analyse de la variance (ou Anova pour Analysis of Variance). Avantageusement, cette analyse peut permettre de vérifier si la valeur moyenne de la variable est significativement différente pour les différentes classes. Cette analyse peut également permettre d'estimer les variances intra-groupes (c'est-à-dire résiduelle) et inter-groupes (c'est-à-dire factorielle). De manière particulièrement avantageuse, la variance factorielle peut permettre de savoir si les valeurs de la variable étudiée évoluent en fonction des groupes.

A l'issue de cette étape de sélection (étape d)), un classifieur peut être généré en apprenant grâce aux données d'entrée recueillies avec les capteurs décrits ci-avant, qui seront classées de façon juste et connues, et à partir des variables sélectionnées Le classifieur est capable de classer correctement les signaux qui seront mesurés sur les capteurs non invasifs susceptibles d'être mis en œuvre à partir de l'étape g), et qui sont avantageusement en nombre inférieurs au nombre de muscles intégrés dans le groupe étudié, en fonction des schémas de co-contraction..

Avantageusement, l'étape suivante d'évaluation de la performance du classifieur par validation croisée et indicateurs de performance (étape e)) peut permettre de réduire les biais d'analyse dû à la taille de la base d'apprentissage. En général pour évaluer les performances d'un algorithme de classification, la base labellisée initiale est divisée en deux sous-ensembles, nommés respectivement la base d'apprentissage et la base de test, la première permettant l'apprentissage de l'algorithme et la seconde servant à la prédiction et à l'évaluation de ces prédictions. Pour avoir des résultats statistiquement significatifs avec cette méthode, la base initiale doit être composée d'un nombre de signaux adapté, que l'homme du métier sait évaluer en fonction de ses connaissances des outils statistiques. Si cette condition initiale n'est pas respectée, il est possible d'utiliser la méthode de validation croisée. En effet, cette dernière consiste à diviser la base de signaux en n sous-ensembles (n>2). Ces sous-ensembles doivent être approximativement de même taille et composés d'une répartition homogène d'individus représentant les différents groupes de classification. Une fois cette répartition effectuée, on utilise (n-1) sous-ensembles pour constituer la base d'apprentissage de l'algorithme et un sous-ensemble pour la base de test. Avantageusement, cette méthode permet de prédire la classe de tous les individus de la base initiale sans utiliser les mêmes signaux dans les bases de d'apprentissage et de test.

Une fois la validation croisée effectuée, un calcul d'indicateurs de performances, tels que la sensibilité, la spécificité et l'indice ROC (Receiver Operating Characteristic), peut être réalisé par tout moyen adapté connu de l'homme du métier.

Par exemple, une matrice de confusion des algorithmes de classification peut être construite. Cette dernière consiste à compter le nombre de vrai positif, de faux positif, de vrai négatif et de faux négatif. A partir de cette matrice de confusion, différents indicateurs de performances peuvent être calculés, tels que la sensibilité, la spécificité et l'indice ROC (Receiver Operating Characteristic). La sensibilité (Se) correspond au taux de vrais positifs, la spécificité (Spe) correspond quant à elle au taux de faux négatifs. La qualité d'un modèle dépend du compromis entre la sensibilité et la spécificité, qui peut être obtenu en calculant l'indice ROC (Receiver Operating Characteristic) avec l'équation suivante :

$$ROC = (1 - Spe)^2 + (1 - Se)^2$$

L'indicateur peut être le taux de bonne classification, qui correspond au taux d'individus bien classé.

Enfin, l'étape g) d'utilisation du classifieur peut être réalisée, notamment par application dudit classifieur à de nouveaux signaux électriques issus d'au moins un capteur dit « cible » placé sur la peau d'un sujet. Avantageusement, une fois le classifieur implémenté de façon optimale (étape d et e), l'information que l'on pourra recueillir sur les co-contraction d'au moins un muscle de surface et au moins un muscle profond peuvent n'être issus que de capteurs « cibles » non invasifs, en nombre inférieur au nombre de muscles étudiés dans le groupement. Avantageusement, le nombre de capteurs « cibles » susceptibles d'être utilisés une fois le classifieur implémenté peut être strictement inférieur au nombre de muscles (ou à la somme du nombre de muscles profonds et de muscles de surface) dont on cherche à caractériser l'activité, tout en étant strictement supérieure à 0. En d'autres termes, si l'on cherche à caractériser l'activité de N muscles, le nombre de capteurs « cibles » suffisant pour mettre en œuvre l'invention est d'au plus N-1. Il peut s'agir d'un nombre compris entre 1 et N-1, en fonction du nombre de muscles à caractériser. Avantageusement, les capteurs « cibles » sont définis dès la mise en œuvre de l'étape a), car ils permettent d'optimiser le classifieurs et de choisir les bonnes variables. Bien sûr, un nombre de capteurs « cible » égal ou supérieur au nombre de muscles à caractériser peut être utilisé, mais cela n'est pas une nécessité pour mettre en œuvre l'invention et obtenir ses avantages.

Toute méthode connue de l'homme du métier peut être utilisée pour l'implémentation. Il peut s'agir par exemple d'au moins une méthode choisie parmi les machines à vecteurs de support (SVM) et le perceptron multicouche (MLP).

La technique du SVM est un ensemble de méthode d'apprentissage supervisée (c'est-à-dire une méthode utilisant un modèle et une base d'apprentissage) qui est utilisée pour résoudre des problèmes de discrimination (à savoir une séparation en classe homogène) et de régression. Cette technique est souvent utilisée en tant que classifieur linéaire. Le principe est le suivant, une fonction h(x), qui est une combinaison linéaire est appelée le noyau. En entrée elle prend un individu et en sortie elle donne une classe. Pour que cette fonction soit capable de déterminer la classe d'un individu, elle apprend en premier lieu à les différencier au moyen d'une base d'apprentissage. La base d'apprentissage contient un ensemble d'individus dont on connaît a priori la classe. Ces individus possèdent des caractéristiques décrites par la fonction h(x). La séparation entre les différents groupes est un hyperplan, situé à h(x) = 0. Si on prend deux classes par exemple, tous les points ayant h(x) > 0, alors ils sont de la classe 1, sinon ils sont placés dans la classe deux. Une fois que la fonction a appris à discriminer les différents groupes, un nouvel individu lui est fourni en entrée, suite à quoi elle renvoit son groupe.

Le perceptron multicouche (MLP) est une sorte de réseau de neurones artificiels. Le MLP peut être constitué de plusieurs couches de neurones et chacune de ces couches d'un nombre variables de neurones. Tous les neurones d'une couche sont reliés aux neurones des couches adjacentes. La liaison entre deux neurones est pondérée par un coefficient. Cette méthode nécessite un apprentissage. Pour faire apprendre le réseau de neurones, les caractéristiques d'individu dont on connaît la classe lui sont données, puis ces caractéristiques passent par chaque couche du perceptron. Une fois le résultat récupéré, le MLP calcule l'erreur entre la vraie classe et la classe prédite, souvent à l'aide d'une moyenne quadratique. Si l'erreur est grande, alors les poids des neurones sont modifiés ; sinon on passe à l'échantillon suivant. Cette méthode permet d'ajuster le réseau de neurones jusqu'à obtenir un bon prédicteur. Une fois l'apprentissage réalisé, on donne en entrée du perceptron les nouveaux échantillons, et ce dernier calcule leurs classes.

Un autre objet de l'invention se rapporte à un produit programme d'ordinateur téléchargeable depuis un réseau de communication et/ou stocké sur un support lisible par ordinateur et/ou exécutable par un microprocesseur, caractérisé en ce qu'il comprend des instructions de code de programme pour l'exécution de la méthode implémentée par ordinateur de classification de signaux physiologiques, lorsqu'il est exécuté sur un ordinateur.

Un autre objet de l'invention se rapporte à une méthode de mesure de l'activité musculaire spécifique d'un groupement de muscles comprenant au moins un muscle profond et au moins un muscle de surface d'un sujet, comprenant les étapes suivantes :

1) acquisition des signaux électriques issus d'au moins un capteur placé sur la peau d'un sujet,

2) pré-traitement des signaux électriques acquis pendant l'étape (1), pour éliminer les bruits parasites et les artéfacts de mouvement du sujet,

3) extraction des variables qui ont été sélectionnées lors de la mise au point du classifieur telle que précédemment décrite (étapes a) à g) de la méthode implémentée par ordinateur de classification de signaux physiologiques issus de l'activité musculaire spécifique d'un groupement de muscles comprenant au moins un muscle profond et au moins un muscle de surface d'un sujet 4) classification des variables extraites à l'étape 3) au moyen du classifieur tel que défini précédemment.

Avantageusement, cette méthode permet de de caractériser un schémas de co-contraction musculaire d'au moins un muscle profond et d'au moins un muscle de surface simultanément.

Les définitions des termes données dans le cadre de la méthode implémentée par ordinateur de classification de signaux physiologiques sont transposables à la méthode de mesure.

Les méthodes de l'invention étant mise en œuvre par ordinateur, elles peuvent impliquer tout dispositif informatique permettant de mettre en œuvre les différentes étapes, et d'exécuter d'autres fonctions logicielles de routine telles que l'enregistrement des données, l'archivage des données, l'affichage, par exemple au moyen d'un moniteur d'ordinateur qui peut afficher les formes d'onde électromyographiques.

Un autre objet de l'invention se rapporte à une utilisation de la méthode de mesure de l'invention, pour une application choisie parmi la rééducation fonctionnelle, le renforcement musculaire à des fins de bien-être et/ou esthétiques, la prévention des pathologies lombaires, rachidiennes, sportives, ou encore pelviennes et le diagnostic fonctionnel.

D'autres avantages pourront encore apparaître à l'homme du métier à la lecture des exemples ci-dessous, illustrés par les figures annexées, donnés à titre illustratif.

**Brève description des figures**

- La figure 1 A et B représente une prise de mesures initiales avec 4 points de mesures via des capteurs (1a, 1b, 1c, 1d, 1e, 1f, 1g, 1h) EMG de surface pour usage future du classifieur sur des capteurs cibles "TR1" (1e, 1f) et "TR2" (1g, 1h) (OE=oblique externe; GD=grand droit; TR=transverse).

- La figure 2 représente trois prises de mesures (A, B et C) avec des capteurs (1a, 1b, 1c, 1d, 1e, 1f, 1g, 1h pour la figure 2A, 1a, 1b, 1g, 1h, 1i, 1j, 1k, 1l pour la figure 2B et 1e, 1f pour la figure 2C) EMG de surface et uniquement pour la prise de mesure dédiée à la paramétrisation du classifieur et la création de la base d'apprentissage. Cette mise en œuvre a été utilisée pour un classifieurs optimisé avec les capteurs cibles TR1 et TR2, et pour un autre classifieur optimisé avec les capteurs cibles L1 et L2.

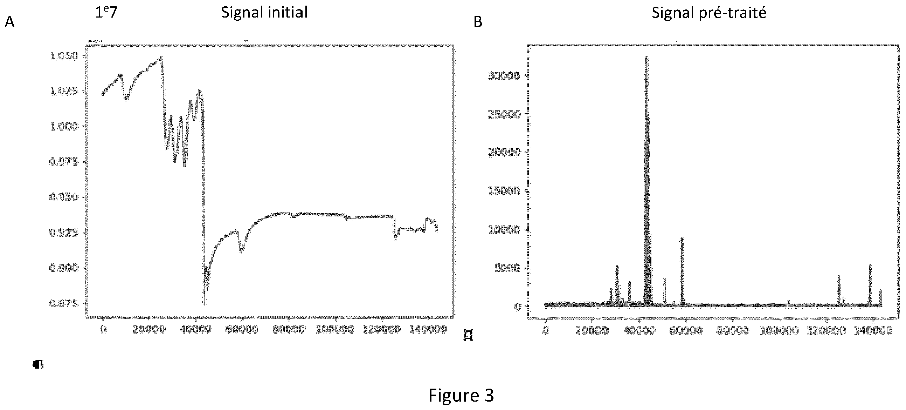

- La figure 3 représente un signal initial (A) et un signal pré-traité (B).

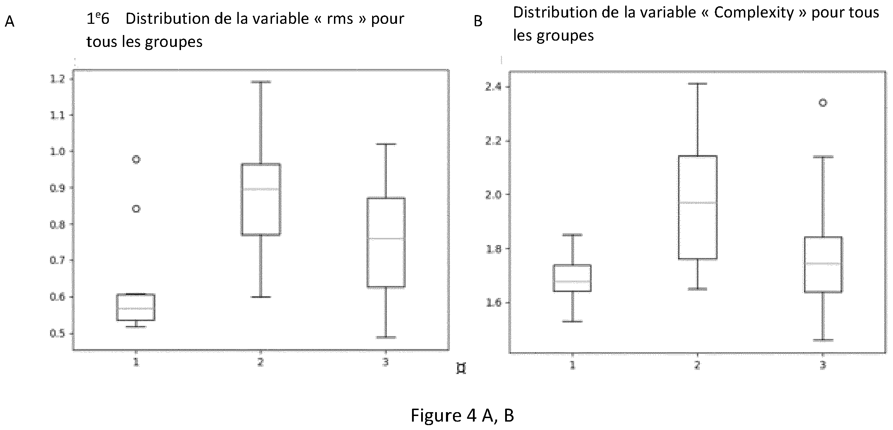

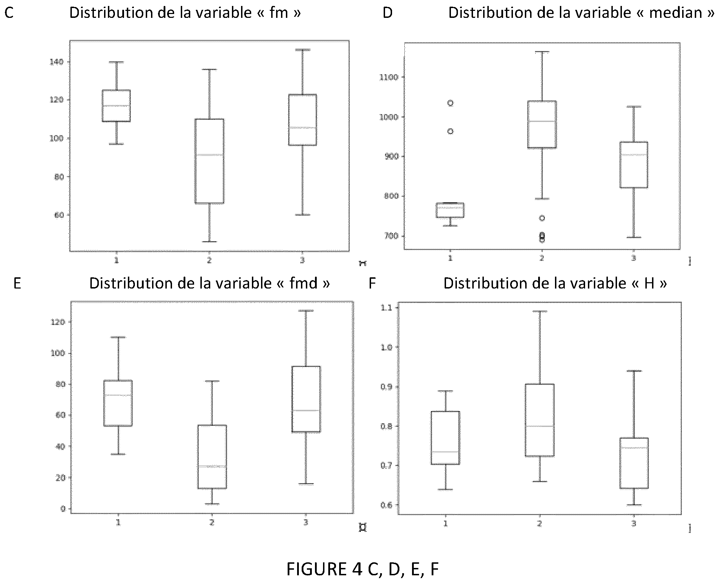

- La figure 4 représente la distribution de la variable « rms » pour tous les groupes (A), la distribution de la variable « Complexity » pour tous les groupes (B), la distribution de la variable « fm » pour tous les groupes (C), la distribution de la variable « median » pour tous les groupes (D), la distribution de la variable « fmd » pour tous les groupes (E), la distribution de la variable « H » pour tous les groupes (F).

- La figure 5 représente les taux de bonne classification pour les classifieurs à 3 variables : [rms, Complexity, fm] (A) et les classifieurs à 4 variables : [median, fm, fmd, H] (B).

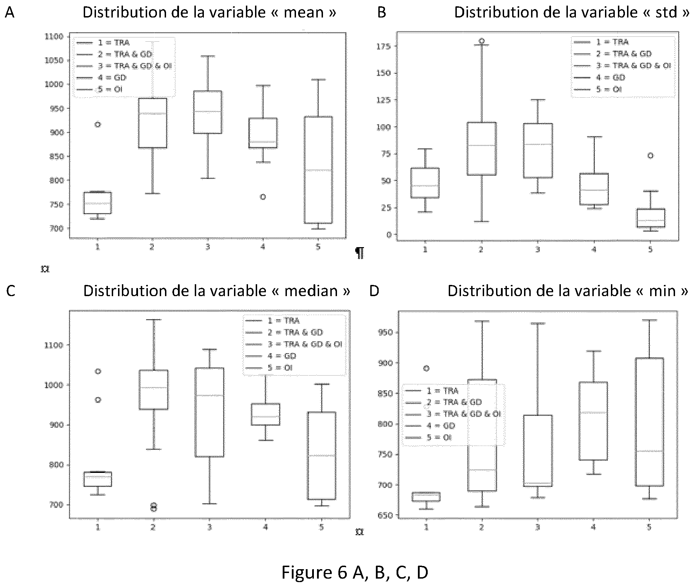

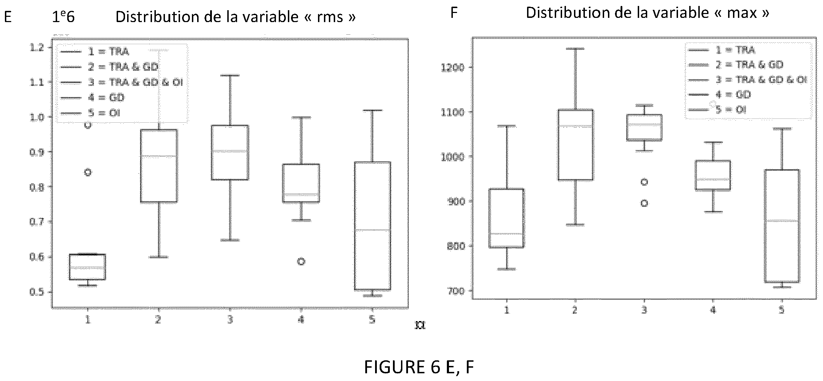

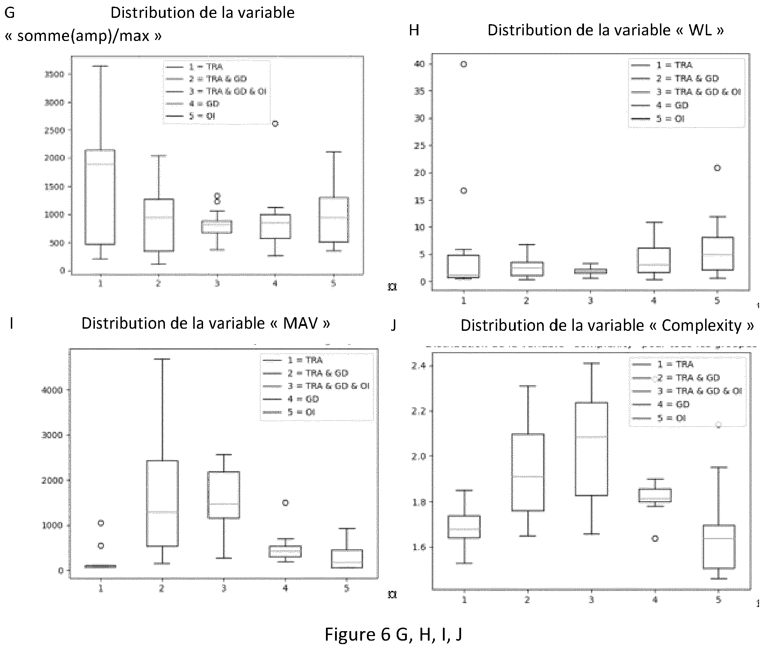

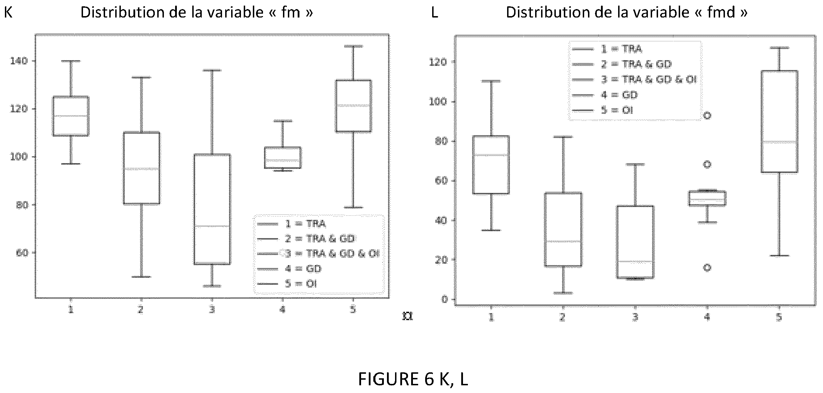

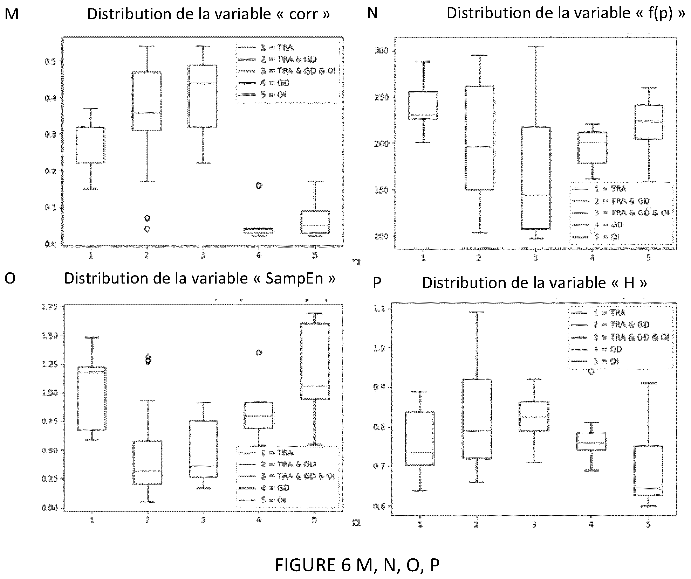

- La figure 6 représente la distribution de la variable « mean » pour tous les groupes (A), la distribution de la variable « std » pour tous les groupes (B), la distribution de la variable « median » pour tous les groupes (C), la distribution de la variable « min » pour tous les groupes (D), la Distribution de la variable « rms » pour tous les groupes (E), la distribution de la variable « max » (F), la Distribution de la variable « somme(amp)/max » pour tous les groupes (G), la distribution de la variable « WL » pour tous les groupes (H), la distribution de la variable « MAV » pour tous les groupes (I), la distribution de la variable « Complexity » pour tous les groupes (J), la distribution de la variable « fm » pour tous les groupes (K), la distribution de la variable « fmd » pour tous les groupes (L), la distribution de la variable « corr » pour tous les groupes (M), la distribution de la variable « f(p) » pour tous les groupes (N), la distribution de la variable « SampEn » pour tous les groupes (O), la distribution de la variable « H » (P).

- La figure 7 représente le taux de bonne classification suite à l'étape de sélection décrite dans l'exemple 4.

- La figure 8 représente la distribution de la variable « fm » pour tous les groupes (A), la distribution de la variable « fmd » pour tous les groupes (B), la distribution de la variable « fp » pour tous les groupes (C), la distribution de la variable « corr » pour tous les groupes (D), la distribution de la variable « wl » pour tous les groupes (E), la distribution de la variable « rms » pour tous les groupes (F).

- La figure 9 (A, B) représente les résultats obtenus ainsi que le taux de bonne classification calculé lors de la mise en œuvre de la méthode décrite dans l'exemple 5.

## EXEMPLES

### Exemple 1 : Exemple de mise en œuvre du recueil des mesures physiologiques

Les mesures sont recueillies sur une zone anatomique comprenant N muscles, dont au moins un muscle profond, à partir de capteurs (1a, 1b, 1c, 1d, 1e, 1f, 1g, 1h) EMG de surface multiples permettant à chaque instant d'avoir l'information sur la contraction ou le relâchement de chaque muscle. Parmi ces capteurs, il y a des capteurs dits "cibles", qui sont des capteurs EMG de surface non invasifs, au nombre de N-1 maximum. Ces capteurs mesurent les signaux qui devront être classifiés par le classifieur mis au point par la méthode.

Le recueil de données permet d'obtenir une base de données de signaux assez fournie pour servir ensuite de base d'apprentissage, et pour permettre de paramétrer de façon optimale le classifieur.

4 jeux de données ont été utilisés. Pour ces données, les signaux sont collectés soit par capteurs EMG de surface, soit par imagerie échographique. La zone anatomique choisie est la sangle abdominale, avec les muscles ciblés parmi le transverse abdominal, les obliques internes, les obliques externes, et les muscles Grands Droits de l'abdomen.

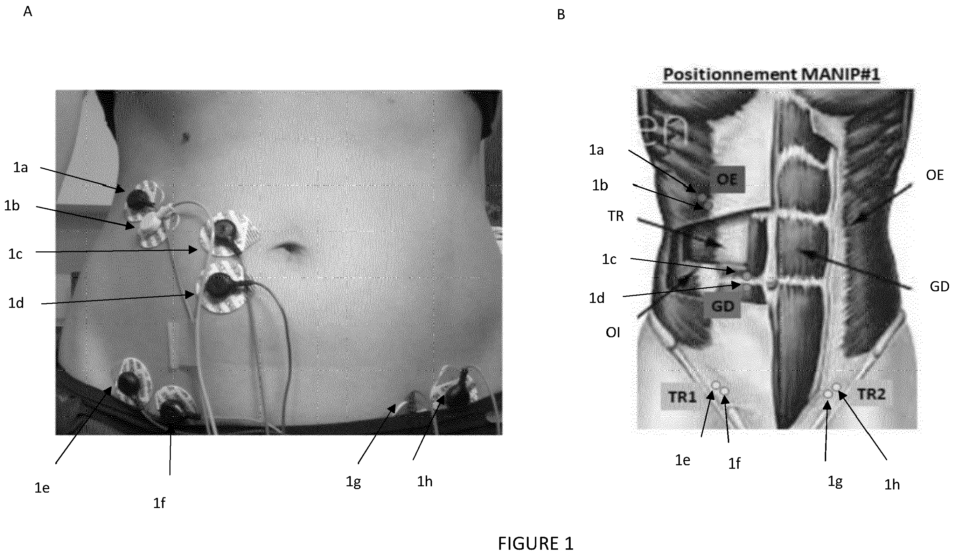

Jeu de données #1: prise de mesures initiales avec 4 points de mesures EMG de surface (1a, 1b, 1c, 1d, 1e, 1f, 1g, 1h) pour usage future du classifieur sur les capteurs dits cibles "TR1" (1e, 1f) et "TR2" (1g, 1h) comme montré sur la figure 1 (OE=oblique externe ; GD=grand droit ; TR=transverse).

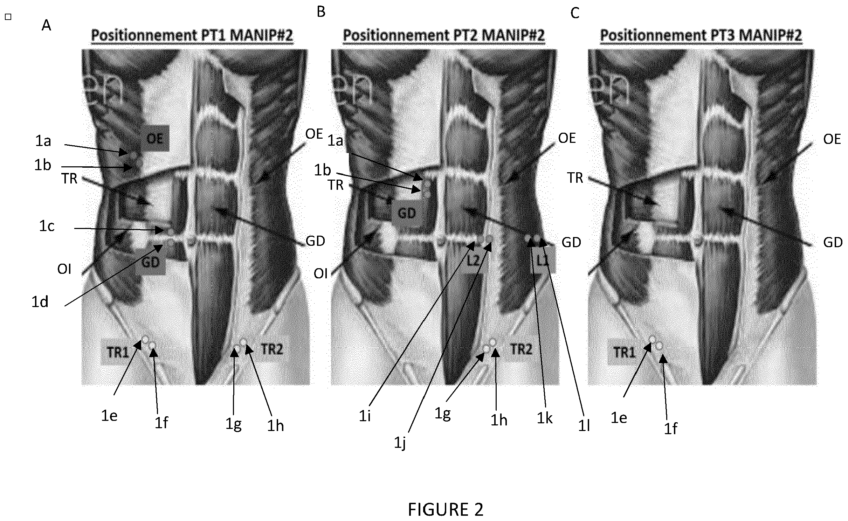

Jeu de données #2: basé à nouveau sur des capteurs EMG de surface uniquement pour la prise de mesure dédiée à la paramétrisation du classifieur et la création de la base d'apprentissage. Trois positionnements différents sont testés comme montré sur la figure 2 A, B et C. Les capteurs dits cibles qui peuvent être utilisés par la suite en entrée du classifieur paramétré par la méthode de l'invention peuvent être les "TR1" et "TR2", ou bien les "L1", ou le "L2".

Jeu de données #3: les mesures initiales sont à nouveau acquises avec des capteurs EMG de surface, placés aux endroits suivants : 2 capteurs sur OED (oblique externe droit), 2 capteurs sur OEG (oblique externe gauche), 2 capteurs sur GDH (grand droit haut), 2 capteurs sur GDB (grand droit bas), 2 capteurs sur OID (oblique interne droit), 2 capteurs sur OIG (oblique interne gauche), 2 capteurs sur LATI (latéral interne), 2 capteurs sur LATE (latéral externe ), sur un premier sujet ; 2 capteurs sur OED, 2 capteurs sur OEG, 2 capteurs sur GDB, 2 capteurs sur OID, 2 capteurs sur OIG, 2 capteurs sur LATI, 2 capteurs sur LATE, 2 capteurs sur MF (moyen fessier) sur un second sujet.

Jeu de données #4: cette fois-ci, les données sont mesurées via des capteurs EMG de surface et via des vidéo échographiques. Les mesures réalisées par imagerie ultrasonore (échographie) sont prises via une sonde linéaire positionnée selon le protocole de l'université de Montréal pour imager la zone abdominale. Les images permettent de mesurer en temps réel les contractions des obliques internes, externes et du transverse abdominal.

### Exemple 2 : Réalisation d'un classifieur

Des travaux préliminaires ont consisté en :

- des analyses exploratoires sur des signaux EMG recueillis par des capteurs EMG de surface placés sur la région basse abdominale, et contenant donc de l'information liée aux contractions des différents muscles « visibles » par les électrodes, à savoir à minima, les Grands Droits, le Muscle Transverse Abdominal et les Obliques Internes. Les analyses exploratoires ont consisté à analyser les caractéristiques temps-fréquences des signaux en fonction des schémas de contractions conjoints ou non de ces trois muscles. Ont été implémenté et analysé :

  ◦ les périodogrammes
  ◦ des décompositions en modes empiriques (EMD),
  ◦ des distributions de Choi-Williams
  ◦ des transformées en ondelettes

- Ces analyses ont permis d'identifier des indicateurs pouvant être de bons candidats pour classifier les signaux en fonction des réponses correspondant à des moments ou le muscle transverse abdominal se contracte et des moments ou le muscle transverse abdominal ne se contracte pas.
- Enfin, plusieurs classifieurs ont été implémentés sur la base de méthodes relevées dans la littérature scientifique. Les indicateurs candidats repérés précédemment ont été paramétrés en entrée des ces classifieurs afin de les tester et de mesurer leur efficacité théorique (par validations croisées sur des données connues).

Deux types de classifieurs ont été envisagés au cours de cette recherche exploratoire, tout d'abord des classifieurs de type SVM (Support Vector Machine), puis des classifieurs de type MLP (MultiLayers Perceptron) (Demont A, Lemarinel M. : « Échographie musculaire de l'abdomen : principes de base et applications cliniques pour la lombalgie commune chronique ». Kinesither Rev. 2017;17(182):41-49 ([7]).

Pour les classifieurs de type SVM, différentes variables d'entrée ont été utilisées, et différents noyaux ont été testés afin de savoir lequel donnait la meilleure spécificité.

La même méthode analytique a été appliquée pour les classifieurs de type MLP, en faisant également varier le nombre de neurones par couche. En ce qui concerne les variables d'entrées, les valeurs singulières extraites de la décomposition en mode empirique ont tout d'abord été utilisées (en suivant une méthode proposée par Lingmei Ai, Jue Wang, Ruoxia Yao (« Classification of parkinsonian and essential tremor using empirical mode décomposition and support vector machine » ([8])).

Suite à cela, les classifieurs ont été alimentés par d'autres indicateurs spectraux et temporels simples, tels que la fréquence moyenne ou la corrélation temporelle entre les deux voies.

Au total, près de 511 configurations différentes ont été testées sur ces classifieurs, et des efficacités de classification de l'ordre de 70% à 80% ont été atteintes.

La méthode a ensuite été optimisée afin d'être utilisable sur n'importe quel jeu de données recueillies et labellisées par mesures EMG de surface. Par labellisées, on entend la capacité à chaque instant et sur chaque voie de mesure, d'indiquer quel(s) muscle(s) sont contractés ou relâché(s). La méthode a alors permis de paramétrer des classifieurs atteignant des efficacités de classification de l'ordre de 96% sur la zone de la sangle abdominale.

Les inventeurs ont réussi à définir d'autres indicateurs que ceux utilisés dans l'art antérieur, permettant d'atteindre des efficacités de classification plus grandes.

En effet, les méthodes utilisées dans l'art antérieur pour mettre au point des classifieurs de signaux EMG consistent souvent à prendre un jeu d'indicateurs "homogènes", c'est à dire que les indicateurs seront tous du même type: soit le classifieur se base sur des indicateurs temporels, soit sur des indicateurs temps-fréquence, soit sur des indicateurs plus exotiques, mais uniquement ceux-ci. Dans la présente invention, les résultats ont été grandement optimisés et améliorés au moment où nous avons choisi un jeu d'indicateurs hybrides, certains provenant d'analyses temporelles, d'autres provenant d'analyses temps-fréquence, ou d'analyse fractale.

**Exemple 3 : Exemple de mise en œuvre du classifieur**

La sangle abdominale est un compartiment multicouches. Elles est composée de :

- une couche de peau et une couche de tissu subcutanée,
- un fascia superficiel et un fascia profond,
- une couche musculaire,
- le fascia transversalis et le péritoine qui protège les viscères.

La couche musculaire de la sangle abdominale est composée de 5 muscles pairs, deux muscles verticaux, le grand droit et le pyramidal, et trois muscles latéraux, l'oblique externe, l'oblique interne et le transverse abdominal. Le rôle principal de ces muscles est de protéger les viscères. Ils génèrent et régulent également la pression intra-abdominale.

Les capteurs mesurant les données qui ont été utilisées sont annoté BBP (sous epine iliaque antéro-supérieure). Ils récupèrent les signaux des muscles suivants : grand droit, oblique externe, oblique interne, muscle transverse abdominal, psoas et périnée.

D'autres signaux ont également été acquis, tels que les signaux du grand droit à l'aide d'électrodes de surface placés selon les directives du SENIAM ((Surface ElectroMyoGraphy for the Non-Invasive Assessment of Muscles). Des vidéos par échographie ont également été acqusies, afin d'améliorer la classification dite experte.

La méthode est suivie pour mettre au point un classifieur qui permet de discriminer trois classes parmi des signaux mesurés par deux électrodes de surface EMG, positionnées sur les emplacements "BBP" (sous epine iliaque antéro-supérieure) - qui ne correspondent donc à aucun positionnement référencé SENIAM. À partir de ce placement, la méthode permet de discriminer le Transverse Abdominal, les Obliques Internes et les Grands Droits.

Pour l'étape 5 (Evaluation de la performance par validation croisee et indicateurs de performance), le classifieur utilisé est un SVM avec un noyau linéaire. La base d'indicateurs utilisés au début de cette analyse était composée des indicateurs suivants: rms, Hj, fm, fmd, médian, H, WL, Sk, M, FuzzyEn

Suite à l'étape n°4 (Sélection des variables), deux jeux d'indicateurs ont été sélectionnés pour implémentation:

Jeu 1: rms, Hj et fm
Jeu 2: médian, fm, fmd, H

La validation croisée est utilisée pour évaluer les performances des classifieurs, en coupant la base d'apprentissage initiale en 5 sous-ensembles.

Matrice de confusion de ['rms','Hj','fm']

| Prédit \| Vrai | 1 | 2 | 3 |
|---|---|---|---|
| 1 | 9 | 1 | 0 |
| 2 | 0 | 8 | 2 |
| 3 | 0 | 2 | 8 |

Matrice de confusion de ['median','fm','fmd','H']

| Prédit \| Vrai | 1 | 2 | 3 |
|---|---|---|---|
| 1 | 2 | 8 | 0 |
| 2 | 1 | 6 | 3 |
| 3 | 0 | 1 | 9 |

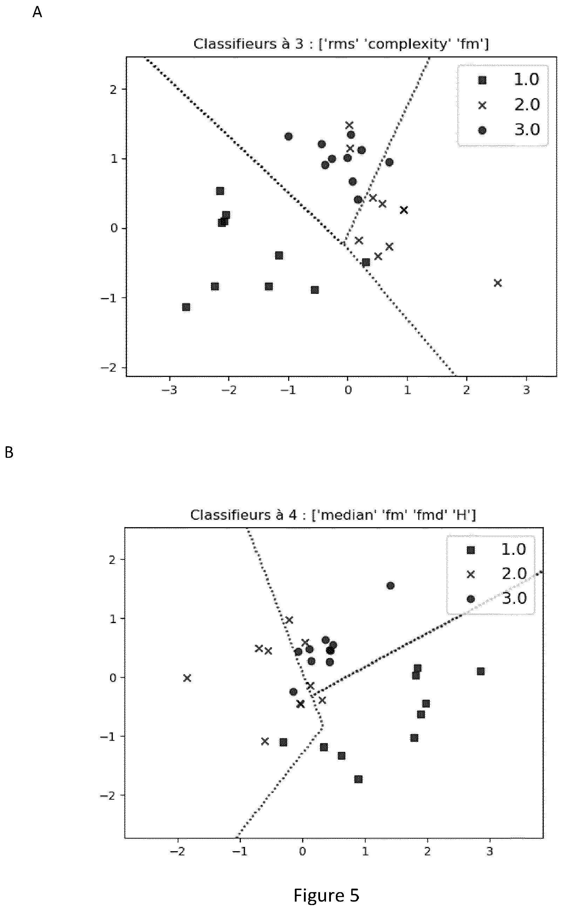

Nous avons obtenus les classifieurs et les taux de bonnes classification montrés en figure 5.

## Exemple 4 : Exemple de mise en œuvre du classifieur

Les signaux utilisés dans cet exemple sont les mêmes que pour l'exemple précédent. Les classifieurs implémentés ici cherchent à discriminer des groupes spécifiques de muscles contractés. Cinq classes sont mises en évidence :

- 1 = TRA
- 2 = TRA & GD
- 3 = TRA & GD & OI
- 4 = GD
- 5 OI

Les indicateurs suivants ont été choisis pour former la base d'apprentissage : mean, std, médian, min, rms, max, somme(amp)/max, wl, MAV, Hj (Complexity), fm, fmd, corr, f(p) et sampEn. La figure 6 montre la répartition de chaque indicateur choisi en fonction des groupes de classification.

Suite à une étape de sélection, le jeu de données suivant a été pris comme base d'apprentissage : Jeu 1 : 'mean' 'std' 'median' 'min' 'rms' 'max' 'MAV' 'Complexity' 'fmd' 'corr' 'f(p)' 'SampEn' 'H'

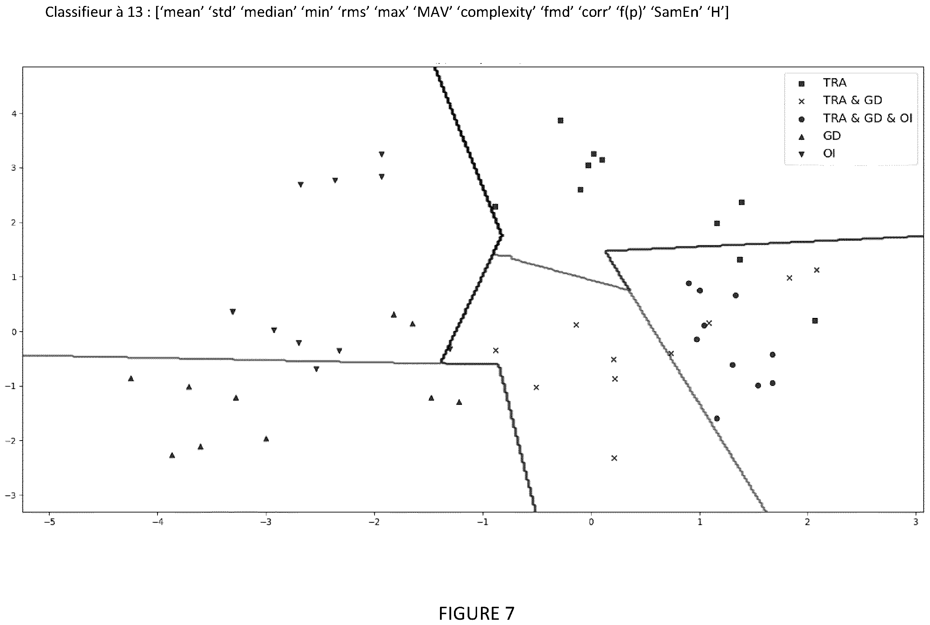

Les taux de bonne classification sont montrés en figure 7.

Matrice de confusion de ['mean' 'std' 'median' 'min' 'rms' 'max' 'MAV' 'Complexity' 'fmd' 'corr' 'f(p)' 'SampEn' 'H']

|   | 1 | 2 | 3 | 4 | 5 |
|---|---|---|---|---|---|
| 1 | 8 | 0 | 2 | 0 | 0 |
| 2 | 0 | 5 | 4 | 1 | 0 |
| 3 | 0 | 2 | 8 | 0 | 0 |
| 4 | 0 | 0 | 0 | 8 | 2 |
| 5 | 0 | 0 | 0 | 5 | 5 |

## Exemple 5 : Exemple de mise en œuvre du classifieur

Pour ce troisième exemple de mise en œuvre du classifieur, les signaux utilisés sont tirés de la collecte de données illustrée en figure 2. La zone étudiée est toujours le caisson abdominal. Toutefois, le placement des électrodes "cibles" diffère des deux exemples précédents. En effet, les capteurs dits cibles pour l'usage futur du classifieur sont ici les capteurs L1 et L2.

Trois classes sont mises en évidence :

1 = TRA
2 = TRA & GD
3 = GD

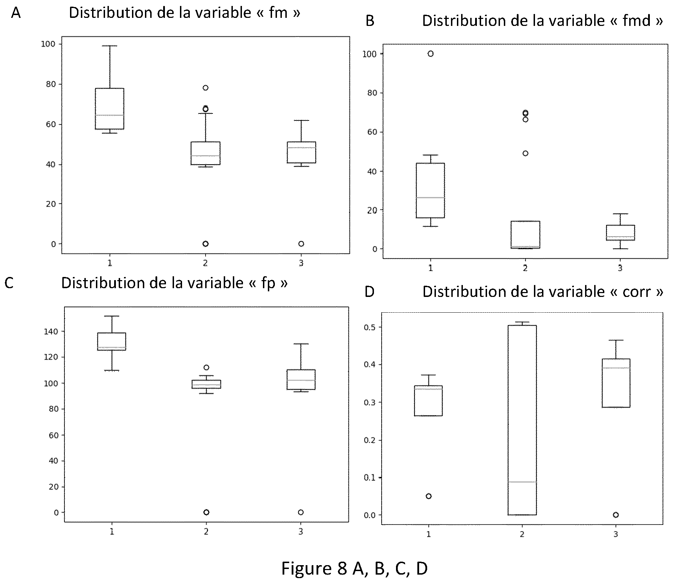

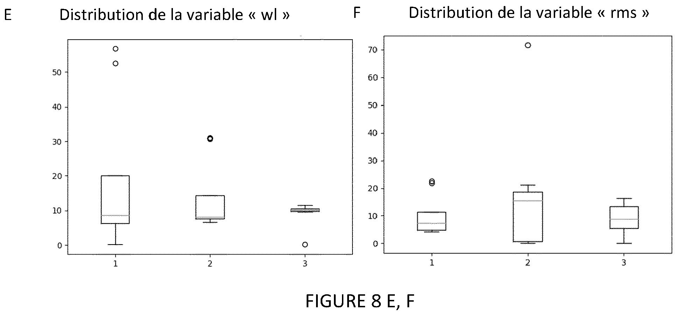

Les indicateurs suivants ont été choisis pour former la base d'apprentissage: somme(amp)/max, wl, Activity, Hj, Skewness, AUC/max, fm, fmd, corr et f(p). La figure 8 montre la répartition d'indicateurs choisis en fonction des groupes de classification.

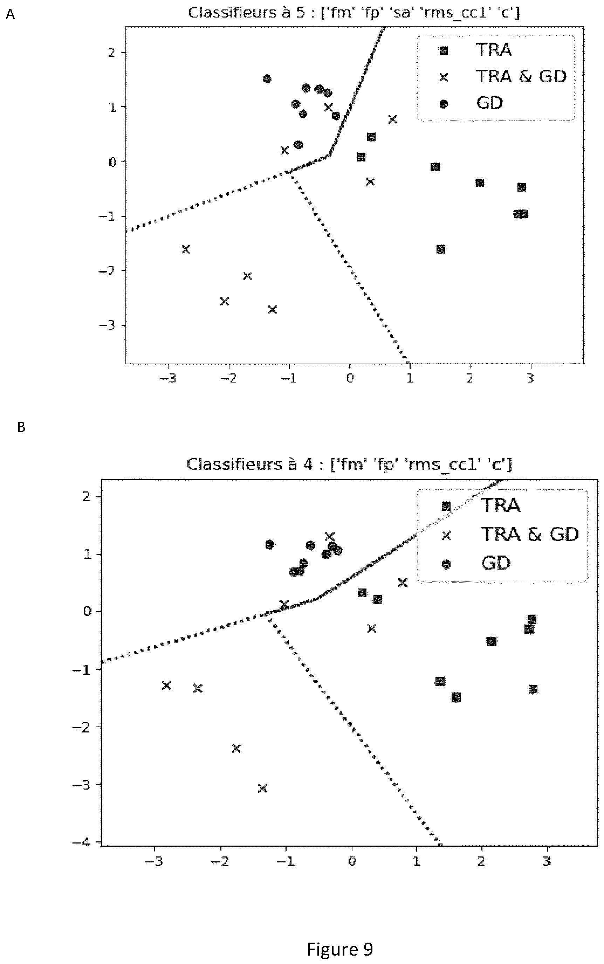

La figure 9 montre les résultats obtenus ainsi que le taux de bonne classification calculé.

## Listes des références

1. McGill et al (1996, J. Biomechanics, Vol 29, No 11, pp 1503-1507.

2. Jesinger et Stonick (1994, 6th IEEE DSP Workshop Proc., p57-60.

3. US 9,687,168.

4. WO2019097166.

5. Fajardo et al.: « EMG hand gesture classification using handcrafted and deep features », Biomedical Signal Processing and Control, Volume 63, January 2021, 102210.

6. Arteaga et al.: « EMG-driven hand model based on the classification of individual finger movements », Biomedical Signal Processing and Control, Volume 58, April 2020, 101834.

7. Demont A, Lemarinel M. : « Échographie musculaire de l'abdomen : principes de base et applications cliniques pour la lombalgie commune chronique ». Kinesither Rev. 2017;17(182):41-49.

8. Lingmei Ai et al. (« Classification of parkinsonian and essential tremor using empirical mode décomposition and support vector machine ». Digital Signal Processing; Volume 21, Issue 4, July 2011, Pages 543-550.

9. Cengiz Tepe, Mehmet Can Demir. The effects of the number of channels and gyroscopic data on the classification performance in EMG data acquired by Myo armband. Journal of Computational Science; Volume 51, April 2021, 101348.

10. Jose Manuel Fajardo, Orlando Gomez, Flavio Prieto : "EMG hand gesture classification using handcrafted and deep features. Biomedical Signal Processing and Control; Volume 63, January 2021, 102210.

11. Noemi Gozzia, Lorenzo Malandri, Fabio Mercorio, Alessandra Pedrocchi : « XAI for myo-controlled prosthesis: Explaining EMG data for hand gesture classification". Knowledge-Based Systems; Volume 240, 15 March 2022, 108053.

12. Firas Sabar Miften, Mohammed Diykh, Shahab Abdulla, Siuly Siuly, Jonathan H.Green, Ravinesh C.Deo: "A new framework for classification of multi-category hand grasps using EMG signais". Artificial Intelligence in Medicine; Volume 112, February 2021, 102005.

13. Maria V.Arteaga, Jenny C.Castiblanco, Ivan F.Mondragon, Julian D.Colorado, Catalina Alvarado-Rojas : "EMG-driven hand model based on the classification of individual finger movements". Biomedical Signal Processing and Control; Volume 58, April 2020, 101834.

14. Abdoulaye Thioune : «Décomposition modale empirique et décomposition spectrale intrinsèque : applications en traitement du signal et de l'image ». Thèse soutenue le 27/09/2016. HAL Id: tel-01372335.

15. Mario Cifrek, Vladimir Medved, Stanko Tonkovic, Sasa Ostoji: "Surface EMG based muscle fatigue evaluation in biomechanics". Clinical Biomechanics; Volume 24, Issue 4, May 2009, Pages 327-340.

16. Lorenz Kahl, Ulrich G. Hofmann: "Comparison of algorithms to quantify muscle fatigue in upper limb muscles based on sEMG signais". Med Eng Phys; 2016 Nov;38(11):1260-1269.

17. Arlene John, Aravind E. Vijayan, A. P. Sudheer: "Electromyography based control of robotic arm using entropy and zero crossing rate". AIR '15: Proceedings of the 2015 Conférence on Advances In RoboticsJuly 2015 Article No.: 69Pages 1-6.

## Revendications

1. Méthode implémentée par ordinateur de classification de signaux physiologiques issus de l'activité musculaire spécifique d'un groupement de muscles comprenant au moins un muscle profond et au moins un muscle de surface d'un sujet, comprenant les étapes suivantes :

   a) acquisition des signaux électriques issus d'au moins un capteur (1) placé sur la peau d'un sujet,
   b) pré-traitement des signaux électriques acquis pendant l'étape a), pour éliminer les bruits parasites et les artéfacts de mouvement du sujet,
   c) extraction d'au moins une variable temporelle, au moins une variable fréquentielle, au moins une variable temps-fréquence, au moins une variable fractale, au moins une variable cepstrale et au moins une variable statistique, sur les signaux pré-traités de l'étape issus de l'étape b),
   d) sélection de variables par analyse de la variance pour classifier des données ultérieures en fonction de classes choisies, les variables sélectionnées formant un classifieur,
   e) implémentation du classifieur à partir des variables sélectionnées à l'étape d),
   f) évaluation de la performance du classifieur par validation croisée et indicateurs de performance,
   g) utilisation du classifieur pour caractériser l'activité musculaire des groupements musculaires.

2. Méthode selon la revendication 1, dans laquelle l'au moins un capteur (1) est sélectionné parmi les capteurs non invasifs comme des capteurs électromyographiques (EMG) ou d'imagerie médicale, des patchs ou des tatouages temporaires électroniques et les capteurs invasifs, comme des patchs munis d'une aiguille.

3. Méthode selon la revendication 1 ou 2, dans laquelle le nombre de capteurs utilisés à l'étape a. est strictement inférieur à la somme du nombre de muscles profonds et de muscles de surface, tout en étant strictement supérieure à 0.

4. Méthode selon l'une quelconque des revendications précédentes, dans laquelle l'au moins une variable fractale est l'exposant de Hurst.

5. Méthode selon l'une quelconque des revendications précédentes, dans laquelle l'au moins une variable cepstrale est choisie parmi les coefficients cepstraux (Cci), les calculs d'indicateurs temporels tels que la moyenne (mean), la médiane (médian), l'écart quadratique (std) ou la moyenne quadratique (rms) sur les coefficients cepstraux (Cci), et les calculs d'indicateurs mathématiques tels que le minimum (min) et le maximum (max) sur les coefficients cepstraux (Cci).

6. Méthode selon l'une quelconque des revendications précédentes, dans laquelle l'au moins une variable statistique est choisie parmi l'entropie de l'échantillon (SampEn), l'entropie floue (FuzzyEn) et l'entropie de Shannon (ShanEn).

7. Méthode selon l'une quelconque des revendications précédentes, dans laquelle :

   - l'au moins une variable temporelle est choisie parmi la longueur de la forme d'onde (WL), la moyenne des amplitudes du signal (MAV), la somme des amplitudes du signal temporel divisée par la valeur maximale du signal (somme(amp)/max), la corrélation temporelle entre deux voies de mesures (corr), les paramètres de Hjorth (Hj) tels que l'activité (A), la mobilité (mobility) ou la complexité (complexity) et l'asymétrie (Sk),
   - l'au moins une variable fréquentielle est choisie parmi la fréquence moyenne (fm), la fréquence médiane (fmd) et la fréquence de coupure (fp), et
   - l'au moins une variable temps-fréquence est choisie parmi la décomposition en mode empirique (EMD) et la fréquence moyenne immédiate DWT (FMI_dwt).

8. Méthode selon l'une quelconque des revendications précédentes, dans laquelle l'analyse de la variance comprend l'analyse de la variance intra-groupes et/ou l'analyse de la variance inter-groupes.

9. Méthode selon l'une quelconque des revendications précédentes, dans laquelle l'implémentation est réalisée par au moins une méthode choisie parmi les machines à vecteurs de support et le perceptron multicouche.

10. Méthode selon l'une quelconque des revendications précédentes, dans laquelle ledit au moins un muscle profond et ledit au moins un muscle de surface est un muscle de la sangle abdominale, un muscle dorsal, un muscle fessier, un muscle du bras, un muscle de l'avant-bras, un muscle de la jambe, un muscle de la cuisse, un muscle de la face, un muscle du cou, un muscle du thorax, un muscle de l'épaule, un muscle du pied.

11. Méthode selon l'une quelconque des revendications précédentes, dans laquelle ledit au moins un muscle profond est choisi parmi le transverse abdominal, le psoas, le carré de lombes, l'oblique interne, l'ilio dorsal, le long dorsal, les intervertébraux, le sus épineux, les muscles du périnée, les multifides, les muscles de la colonne vertébrale, notamment qui relient l'apophyse épineuse et les apophyses transverses de chaque vertèbre, et la musculature autochtone du dos.

12. Méthode selon l'une quelconque des revendications précédentes, dans laquelle ledit au moins un muscle de surface est choisi parmi l'oblique externe, le grand droit, les muscles scalènes, le muscle sterno-cléido-mastoïdien, les trapèzes, le grand pectoral, les deltoïdes, le grand dorsal, les muscles intercostaux, les biceps, les triceps, les fléchisseurs de l'avant-bras, ou les extenseurs de l'avant-bras, les fessiers, les abducteurs, les adducteurs, les ischio-jambiers, les quadriceps et les jumeaux.

13. Méthode de mesure de l'activité musculaire spécifique d'un groupement de muscles comprenant au moins un muscle profond et au moins un muscle de surface d'un sujet, comprenant les étapes suivantes :

1) acquisition des signaux électriques issus d'au moins un capteur (1) placé sur la peau d'un sujet,
2) pré-traitement des signaux électriques acquis pendant l'étape (a), pour éliminer les bruits parasites et les artéfacts de mouvement du sujet,
3) extraction d'au moins une variable temporelle, au moins une variable fréquentielle, au moins une variable temps-fréquence, au moins une variable fractale, au moins une variable cepstrale et au moins une variable statistique, sur les signaux pré-traités de l'étape issus de l'étape (b),
4) classification des variables extraites à l'étape 3) au moyen du classifieur tel que défini dans l'une quelconque des revendications 1 à 12.

14. Utilisation d'une méthode de mesure selon la revendication 13, pour une application choisie parmi la rééducation fonctionnelle, le renforcement musculaire à des fins de bien-être et/ou esthétiques, la prévention des pathologies lombaires, rachidiennes, sportives, ou encore pelviennes et le diagnostic fonctionnel.

15. Produit programme d'ordinateur téléchargeable depuis un réseau de communication et/ou stocké sur un support lisible par ordinateur et/ou exécutable par un microprocesseur, **caractérisé en ce qu'**il comprend des instructions de code de programme pour l'exécution de la méthode selon l'une au moins des revendications 1 à 11, lorsqu'il est exécuté sur un ordinateur.

FIGURE 1

FIGURE 2

Figure 3

A  1e6  Distribution de la variable « rms » pour tous les groupes

B  Distribution de la variable « Complexity » pour tous les groupes

Figure 4 A, B

C — Distribution de la variable « fm »
D — Distribution de la variable « median »
E — Distribution de la variable « fmd »
F — Distribution de la variable « H »

FIGURE 4 C, D, E, F

A

Classifieurs à 3 : ['rms' 'complexity' 'fm']

B

Classifieurs à 4 : ['median' 'fm' 'fmd' 'H']

Figure 5

Figure 6 A, B, C, D

FIGURE 6 E, F

EP 4 230 141 A1

G Distribution de la variable
« somme(amp)/max »

H Distribution de la variable « WL »

I Distribution de la variable « MAV »

J Distribution de la variable « Complexity »

Figure 6 G, H, I, J

EP 4 230 141 A1

K      Distribution de la variable « fm »

L      Distribution de la variable « fmd »

FIGURE 6 K, L

M  Distribution de la variable « corr »

N  Distribution de la variable « f(p) »

O  Distribution de la variable « SampEn »

P  Distribution de la variable « H »

FIGURE 6 M, N, O, P

Classifieur à 13 : ['mean' 'std' 'median' 'min' 'rms' 'max' 'MAV' 'complexity' 'fmd' 'corr' 'f(p)' 'SamEn' 'H']

FIGURE 7

Figure 8 A, B, C, D

E    Distribution de la variable « wl »

F    Distribution de la variable « rms »

FIGURE 8 E, F

A

Classifieurs à 5 : ['fm' 'fp' 'sa' 'rms_cc1' 'c']

B

Classifieurs à 4 : ['fm' 'fp' 'rms_cc1' 'c']

Figure 9

Europäisches
Patentamt

European
Patent Office

Office européen
des brevets

# RAPPORT DE RECHERCHE EUROPEENNE

Numéro de la demande

EP 22 30 6332

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | MARRI KIRAN ET AL: "Classification of muscle fatigue using surface electromyography signals and multifractals", 2015 12TH INTERNATIONAL CONFERENCE ON FUZZY SYSTEMS AND KNOWLEDGE DISCOVERY (FSKD), IEEE, 15 août 2015 (2015-08-15), pages 669-674, XP032849583, DOI: 10.1109/FSKD.2015.7382022 * page 669 - page 673 * ----- | 1-15 | INV. A61B5/397 A61B5/00 |
| A | SEGUNA CLIVE ET AL: "Development of a New Low-Cost EMG Monitoring System for the Classification of Finger Movement", 2018 NEW GENERATION OF CAS (NGCAS), IEEE, 20 novembre 2018 (2018-11-20), pages 126-129, XP033472646, DOI: 10.1109/NGCAS.2018.8572217 * page 128 * ----- | 1-15 | |
| A | MOQADAM SAEED BAHRAMI ET AL: "A Novel Hybrid Approach to Pattern Recognition of Finger Movements and Grasping Gestures in Upper Limb Amputees", IEEE SENSORS JOURNAL, IEEE, USA, vol. 22, no. 3, 24 décembre 2021 (2021-12-24), pages 2591-2602, XP011899625, ISSN: 1530-437X, DOI: 10.1109/JSEN.2021.3138386 [extrait le 2022-01-28] * page 2593 * ----- | 1-15 | **DOMAINES TECHNIQUES RECHERCHES (IPC)** A61B |
| A | CN 107 126 303 A (SHANGHAI SHULI INTELLIGENT TECH CO LTD) 5 septembre 2017 (2017-09-05) * alinéa [0007] - alinéa [0017] * ----- | 1-15 | |

Le présent rapport a été établi pour toutes les revendications

| Lieu de la recherche | Date d'achèvement de la recherche | Examinateur |
|---|---|---|
| La Haye | 15 mai 2023 | Chau, Thoi Dai |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

    ...................................................................

& : membre de la même famille, document correspondant

EPO FORM 1503 03.82 (P04C02)

**EP 4 230 141 A1**

## ANNEXE AU RAPPORT DE RECHERCHE EUROPEENNE
## RELATIF A LA DEMANDE DE BREVET EUROPEEN NO.

EP 22 30 6332

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

15-05-2023

| Document brevet cité au rapport de recherche | Date de publication | Membre(s) de la famille de brevet(s) | Date de publication |
|---|---|---|---|
| CN 107126303 A | 05-09-2017 | AUCUN | |

EPO FORM P0460

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- US 9687168 B

- WO 2019097166 A

### Littérature non-brevet citée dans la description

- **MCGILL et al.** *J. Biomechanics,* 1996, vol. 29 (11), 1503-1507
- **JESINGER ; STONICK.** *6th IEEE DSP Workshop Proc.,* 1994, 57-60
- **FAJARDO et al.** EMG hand gesture classification using handcrafted and deep features. *Biomedical Signal Processing and Control,* Janvier 2021, vol. 63, 102210
- **ARTEAGA et al.** EMG-driven hand model based on the classification of individual finger movements. *Biomedical Signal Processing and Control,* Avril 2020, vol. 58, 101834
- **DEMONT A ; LEMARINEL M.** Échographie musculaire de l'abdomen : principes de base et applications cliniques pour la lombalgie commune chronique. *Kinesither Rev.,* 2017, vol. 17 (182), 41-49
- **DEMONT A ; LEMARINEL M.** Échographie musculaire de l'abdomen : principes de base et applications cliniques pour la lombalgie commune chronique. *Kinesither Rev.,* 2017, vol. 17 (182), 41-49
- **LINGMEI AI et al.** Classification of parkinsonian and essential tremor using empirical mode décomposition and support vector machine. *Digital Signal Processing,* Juillet 2011, vol. 21 (4), 543-550
- **CENGIZ TEPE ; MEHMET CAN DEMIR.** The effects of the number of channels and gyroscopic data on the classification performance in EMG data acquired by Myo armband. *Journal of Computational Science,* Avril 2021, vol. 51, 101348
- **JOSE MANUEL FAJARDO ; ORLANDO GOMEZ ; FLAVIO PRIETO.** EMG hand gesture classification using handcrafted and deep features. *Biomedical Signal Processing and Control,* Janvier 2021, vol. 63, 102210

- **NOEMI GOZZIA ; LORENZO MALANDRI ; FABIO MERCORIO ; ALESSANDRA PEDROCCHI.** XAI for myo-controlled prosthesis: Explaining EMG data for hand gesture classification. *Knowledge-Based Systems,* 15 Mars 2022, vol. 240, 108053
- **FIRAS SABAR MIFTEN ; MOHAMMED DIYKH ; SHAHAB ABDULLA ; SIULY SIULY ; JONATHAN H.GREEN ; RAVINESH C.DEO.** A new framework for classification of multi-category hand grasps using EMG signais. *Artificial Intelligence in Medicine,* Février 2021, vol. 112, 102005
- **MARIA V.ARTEAGA ; JENNY C.CASTIBLANCO ; IVAN F.MONDRAGON ; JULIAN D.COLORADO ; CATALINA ALVARADO-ROJAS.** EMG-driven hand model based on the classification of individual finger movements. *Biomedical Signal Processing and Control,* Avril 2020, vol. 58, 101834
- **ABDOULAYE THIOUNE.** *Décomposition modale empirique et décomposition spectrale intrinsèque : applications en traitement du signal et de l'image,* 27 Septembre 2016
- **MARIO CIFREK ; VLADIMIR MEDVED ; STANKO TONKOVIC ; SAŠA OSTOJI.** Surface EMG based muscle fatigue evaluation in biomechanics. *Clinical Biomechanics,* Mai 2009, vol. 24 (4), 327-340
- **LORENZ KAHL ; ULRICH G. HOFMANN.** Comparison of algorithms to quantify muscle fatigue in upper limb muscles based on sEMG signais. *Med Eng Phys,* Novembre 2016, vol. 38 (11), 1260-1269
- **ARLENE JOHN ; ARAVIND E. VIJAYAN ; A. P. SUDHEER.** Electromyography based control of robotic arm using entropy and zero crossing rate. *AIR '15: Proceedings of the 2015 Conférence on Advances In Robotics,* Juillet 2015, (69), 1-6